# EUROPEAN PATENT APPLICATION

(11) **EP 2 923 717 A1**
(43) Date of publication of application: **30.09.2015**
(21) Application number: 14162456.9
(22) Date of filing: 28.03.2014
(51) Int. Cl.: A61M 5/32, A61M 5/31

(54) **Safety needle assembly**

(71) Applicant: Sanofi-Aventis Deutschland GmbH, 65929 Frankfurt am Main (DE)
(72) Inventor: The designation of the inventor has not yet been filed

(57) **Abstract**

The invention relates to a needle assembly (1) comprising:
- a needle hub (2) adapted to be connected to a distal end of a medicament cartridge or cartridge holder (3),
- a hollow needle (4) attached to the needle hub (2), the hollow needle (4) comprising a distal pointed tip (4.1),
- a needle catch arm (5) attached to the needle hub (2) and adapted to be tilted toward the needle (4) for covering the distal pointed tip (4.1).

## Description

### Technical Field

The invention relates to a needle assembly.

### Background of the Invention

Conventional injection devices are typically adapted to be connected to single-use needle assemblies. Typically, a user will attach a needle assembly to the injection device (e.g., by a threaded connection) and remove the needle assembly after an injection has been performed. During removal of the needle assembly from the injection device the user is subjected to the risk of needle stick injury. Various devices have been proposed to reduce this risk.

There remains a need for an improved needle assembly.

### Summary of the Invention

It is an object of the present invention to provide an improved needle assembly.

The object is achieved by a needle assembly according to claim 1.

Exemplary embodiments of the invention are given in the dependent claims.

In an exemplary embodiment a needle assembly according to the invention comprises:
- a needle hub adapted to be connected to a distal end of a medicament cartridge,
- a hollow needle attached to the needle hub, the hollow needle comprising a distal pointed tip,
- a needle catch arm attached to the needle hub and adapted to be tilted toward the needle for covering the distal pointed tip.

In an exemplary embodiment the needle catch arm may in particular be arranged to be tilted between a neutral position, e. g. substantially in parallel with the needle, a medicament delivery position, in which the needle catch arm is tilted outward from the neutral position thus facilitating insertion of the needle into an injection site, and a needle safe position, in which the needle catch arm is tilted toward the needle for covering the distal pointed tip of the needle. In an exemplary embodiment a user may bring the needle catch arm from the neutral or delivery position into the needle safe position by pressing the needle catch arm against a surface, e. g. a table. The user, a patient or caregiver thus avoids coming near the distal pointed tip of the needle with his fingers thus reducing the risk of post injection needle stick injury.

In an exemplary embodiment the needle catch arm extends from the needle hub in a distal direction in the neutral position, wherein a protective needle sheath is removably arrangeable on the needle hub radially inward of the needle catch arm for covering the needle prior to use. In an exemplary embodiment, in order to prepare the needle assembly for an injection after having attached the needle assembly to a medicament cartridge the user may grip the needle catch arm and tilt it from the neutral position towards the delivery position while the protective needle sheath is in place over the needle. The protective needle sheath may then be pulled off the needle in a distal direction rendering the needle assembly ready to be used.

In an exemplary embodiment the needle catch arm is connected to the needle hub by at least one live hinge adapted to allow for tilting the needle catch arm toward and away from the needle. A live hinge is a particularly inexpensive way for allowing relative movement of components connected by the live hinge, in particular if the live hinge is intended to be used a limited number of times as is the case in a single use needle assembly. Components connected by a live hinge may be integrally formed, e. g. injection molded.

In an exemplary embodiment the protective needle sheath comprises an annular proximal collar, wherein at least one of the live hinges is arranged to form a slot at a proximal end of the needle catch arm, wherein the annular proximal collar is adapted to engage in the slot. The protective needle sheath and the needle catch arm thus have corresponding features interacting in such a way that the needle catch arm is tilted from the neutral position towards the delivery position by removing the protective needle sheath.

In an exemplary embodiment the needle catch arm comprises a recess for receiving the needle, wherein the recess is limited by a distal end wall arranged to catch the distal pointed tip of the needle. When the needle catch arm is tilted into the needle safe position the needle is received within the recess. The distal end wall prevents the distal pointed tip of the needle from protruding beyond the distal end of the needle catch arm thus improving post injection needle safety.

In an exemplary embodiment the needle catch arm is arranged to be tilted toward the needle to such an extent that the needle is plastically deformed, e.g. bent by the needle catch arm. Thus, the needle is clearly marked as used thus discouraging the user from attempting to re-tilt the needle catch arm into the delivery position and to re-use the needle. In an exemplary embodiment the distal end wall limiting the recess within the needle catch arm engages the distal pointed tip of the needle while the needle catch arm is being tilted towards the needle safe position and the needle is bent within the recess. This results in the needle being slightly tensioned within the recess such that the distal pointed tip may ingress into the material of the distal end wall further improving post injection needle safety. The material of the distal end wall may be particularly soft for improving this effect.

In an exemplary embodiment the needle assembly further comprises a needle cap removably arrangeable over the needle hub for covering the needle and the needle catch arm. This may allow the user to firmly grip the needle assembly when attaching it to and/or removing it from a medicament cartridge. The needle cap may comprise grip features such as corrugations for improving ergonomics.

In an exemplary embodiment the needle cap may comprise a longitudinal slot having a width at least as great as a width of the needle catch arm. This allows for replacing the needle cap after use of the needle assembly and after the needle catch arm has been tilted into the needle safe position bending the needle to such an extent that the needle catch arm protrudes radially beyond the needle hub. After re-attaching the needle cap the needle catch arm will thus protrude radially through the longitudinal slot providing a clear indication that the needle assembly has been used discouraging a user from attempting to re-use it.

In an exemplary embodiment a sterility tab may be removably arranged over the longitudinal slot prior to use indicating an unused needle assembly when intact. The user will have to peel the sterility tab off the longitudinal slot in order to re-attach the needle cap on the needle hub after the needle catch arm has been tilted into the needle safe position bending the needle. The sterility tab may extend over both the longitudinal slot and a proximal opening of the needle hub thus keeping the entire needle assembly sterile prior to use. The user will then have to peel the sterility off in order to be able to attach the needle hub to a medicament cartridge.

In an exemplary embodiment the needle hub comprises a screw thread adapted to engage a corresponding screw thread on a medicament cartridge. The screw thread may be arranged on an inner surface of a cavity within the needle hub. Likewise, the screw thread may be on an outer surface of the needle hub and the medicament cartridge may have a corresponding thread on an inner surface. In other embodiments the needle hub and the medicament cartridge may comprise corresponding bayonet features or they may have conical surfaces for frictionally fitting the needle hub to the medicament cartridge.

In an exemplary embodiment the needle may further comprise a proximal pointed tip extending within a cavity of the needle hub. This allows for establishing a fluid communication between the needle and the medicament cartridge by the proximal pointed tip piercing a septum on the cartridge while the needle assembly is being attached to the medicament cartridge.

In an exemplary embodiment the needle catch arm and the needle hub may comprise corresponding locking features locking the needle catch arm in the needle safe position thus preventing re-exposure of the needle.

Further scope of applicability of the present invention will become apparent from the detailed description given hereinafter. However, it should be understood that the detailed description and specific examples, while indicating exemplary embodiments of the invention, are given by way of illustration only, since various changes and modifications within the spirit and scope of the invention will become apparent to those skilled in the art from this detailed description.

### Brief Description of the Drawings

The present invention will become more fully understood from the detailed description given hereinbelow and the accompanying drawings which are given by way of illustration only, and thus, are not limitive of the present invention, and wherein:
- Figure 1: is a schematic view of an exemplary embodiment of a needle assembly according to the present invention prior to use,
- Figure 2: is a schematic view of the needle assembly during use,
- Figure 3: is a schematic view of the needle assembly during use,
- Figure 4: is a schematic view of the needle assembly during use,
- Figure 5: is a schematic view of another exemplary embodiment of a needle assembly according to the present invention prior to use,
- Figure 6: is a schematic view of the needle assembly during use,
- Figure 7: is a schematic view of the needle assembly during use,
- Figure 8: is a schematic view of the needle assembly during use,
- Figure 9: is a schematic view of the needle assembly during use, and
- Figure 10: is a schematic view of the needle assembly during use.

Corresponding parts are marked with the same reference symbols in all figures.

### Detailed Description

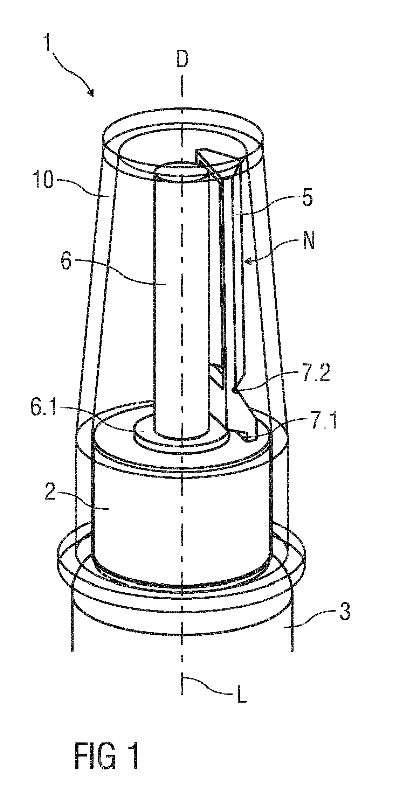

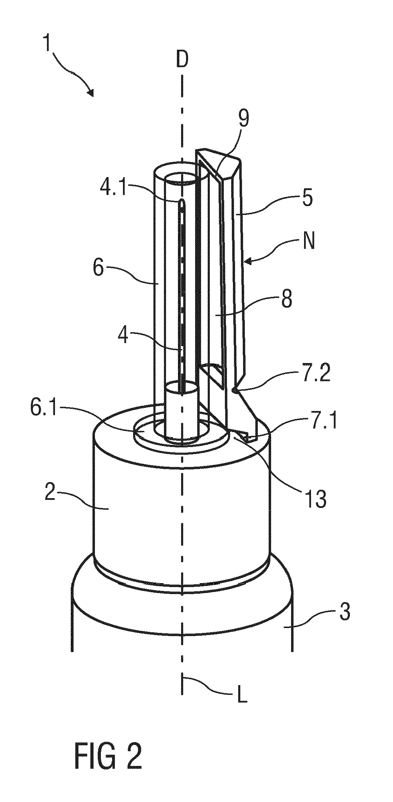

Figure 1 is a schematic transparent view of an exemplary embodiment of a needle assembly 1 according to the present invention prior to use. In an exemplary embodiment the needle assembly 1 comprises:
- a needle hub 2 adapted to be connected to a distal end of a medicament cartridge or a cartridge holder 3 for a medicament cartridge,
- a hollow needle 4 (cf. fig. 2) attached to the needle hub 2, the hollow needle 4 comprising a distal pointed tip 4.1,
- a needle catch arm 5 attached to the needle hub 2 and adapted to be tilted toward the needle 4 for covering the distal pointed tip 4.1.

In an exemplary embodiment the needle catch arm 5 extends from the needle hub 2 in a distal direction D in a neutral position N, e. g. substantially in parallel with the needle 4, wherein a substantially tubular protective needle sheath 6 is removably arranged on the needle hub 2 radially inward of the needle catch arm 5 with respect to a longitudinal axis L for covering the needle 4 prior to use. The protective needle sheath 6 comprises an annular proximal collar 6.1 having a greater diameter than the tubular part of the protective needle sheath 6.

In an exemplary embodiment the needle catch arm 5 is connected to the needle hub 2 by a first live hinge 7.1 which is arranged such that in the neutral position N a tangential slot 13 is formed at a proximal end of the needle catch arm 5 radially inward of the first live hinge 7.1, wherein the annular proximal collar 6.1 of the protective needle sheath 6 engages in the slot 13 such that the protective needle sheath 6 cannot be moved in the distal direction D without tilting the needle catch arm 5 away from the needle 4.

In an exemplary embodiment a second live hinge 7.2 is arranged distally from the first live hinge 7.1 adapted to allow for tilting the catch arm 5 toward the needle 4.

In an exemplary embodiment the needle catch arm 5 comprises a recess 8 for receiving the needle 4, wherein the recess 8 is limited by a distal end wall 9 arranged to catch the distal pointed tip 4.1 of the needle 4.

In an exemplary embodiment the needle assembly 1 further comprises a needle cap 10 removably arranged over the needle hub 2 for covering the needle 4 and the needle catch arm 5.

In figure 1 the needle assembly 1 is in a state as delivered prior to use. The protective needle sheath 6 is in place over the needle 4, the needle catch arm 5 is in the neutral position N and the needle cap 10 is arranged over the needle hub 2, the protective needle sheath 6 and the needle catch arm 5.

In an exemplary embodiment, in order to prepare the needle assembly 1 for an injection the needle assembly 1 is attached to a distal end of a cartridge holder 3 or medicament cartridge. The needle cap 10 may then be gripped and pulled in the distal direction D off the needle hub 2 thus exposing the needle catch arm 5 and the protective needle sheath 6. This state of the needle assembly 1 is illustrated in figure 2.

The protective needle sheath 6 may then be pulled off the needle 4 in the distal direction D exposing the needle 4 and rendering the needle assembly 1 ready to be used for an injection. Due to the annular collar 6.1 engaged in the slot 13 the needle catch arm 5 is tilted about the first live hinge 7.1 from the neutral position N towards a medicament delivery position MD when removing the protective needle sheath 6.

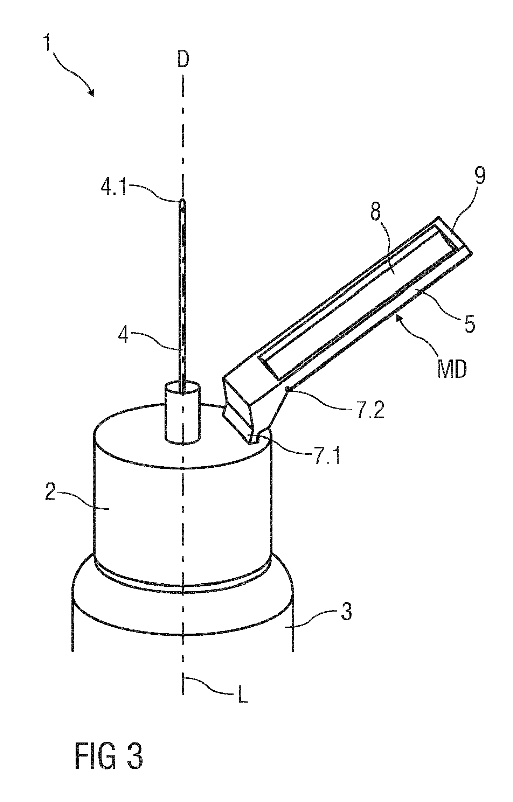

Figure 3 shows the needle assembly 1 with the needle catch arm 5 in the medicament delivery position MD, in which the needle catch arm 5 is tilted radially outward from the neutral position N thus facilitating insertion of the needle 4 into an injection site.

The needle 4 may now be inserted into an injection site and a medicament may be displaced from the medicament cartridge through a lumen in the needle 4.

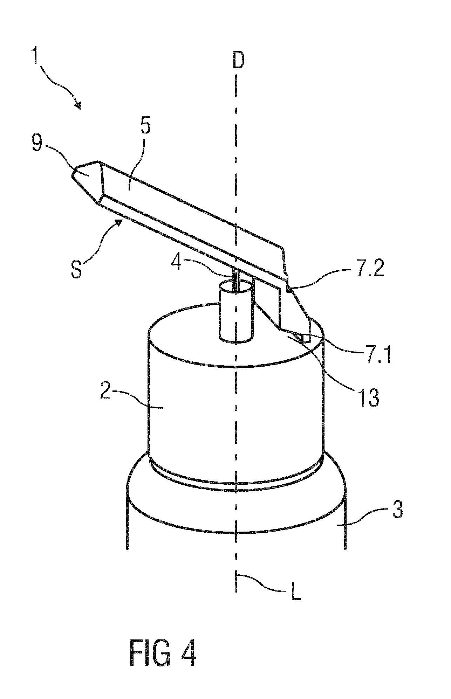

Subsequently or at any other point in time the needle catch arm 5 may be brought into a needle safe position S in which the needle 4 can no longer be accessed. Figure 4 shows the needle assembly 1 with the needle catch arm 5 in the needle safe position S, in which the needle catch arm 5 is tilted toward the needle 4 for covering the distal pointed tip 4.1 of the needle 4. In an exemplary embodiment a user may bring the needle catch arm 5 from the neutral N or delivery position MD into the needle safe position S by pressing the needle catch arm 5 against a surface, e. g. a table.

When the needle catch arm 5 is tilted into the needle safe position S the needle 4 is received within the recess 8. The distal end wall 9 prevents the distal pointed tip 4.1 of the needle 4 from protruding beyond the distal end of the needle catch arm 5.

In an exemplary embodiment the needle catch arm 5 is arranged to be tilted toward the needle 4 to such an extent that the needle 4 is plastically deformed, e.g. bent by the needle catch arm 5. In an exemplary embodiment the distal end wall 9 limiting the recess 8 within the needle catch arm 5 engages the distal pointed tip 4.1 of the needle 4 while the needle catch arm 5 is being tilted towards the needle safe position S and the needle 4 is bent within the recess 8. This results in the needle 4 being at least slightly tensioned within the recess 8 such that the distal pointed tip 4.1 may ingress into the material of the distal end 9 wall further improving post injection needle safety. The material of the distal end wall 9 may be particularly soft for improving this effect.

The needle assembly 1 may now be removed from the cartridge holder 3 or medicament cartridge and disposed.

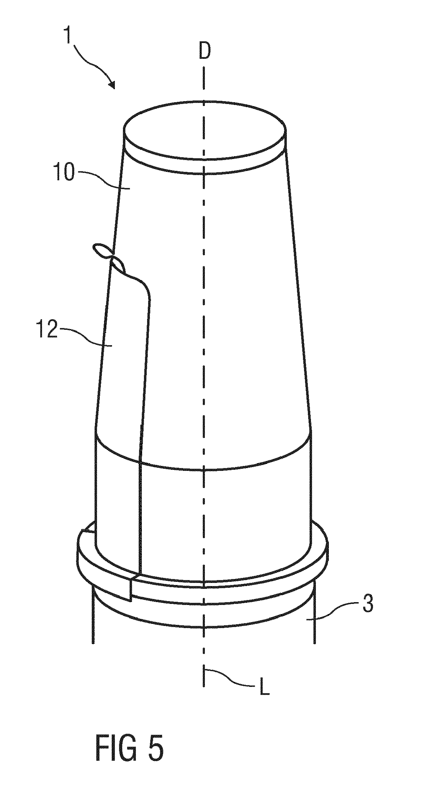

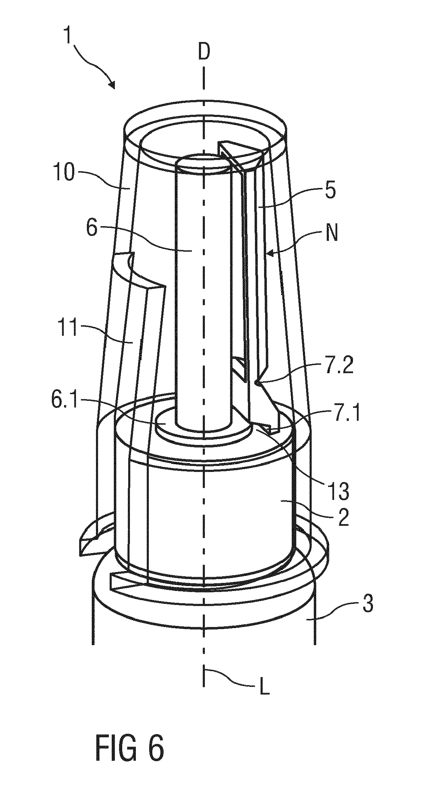

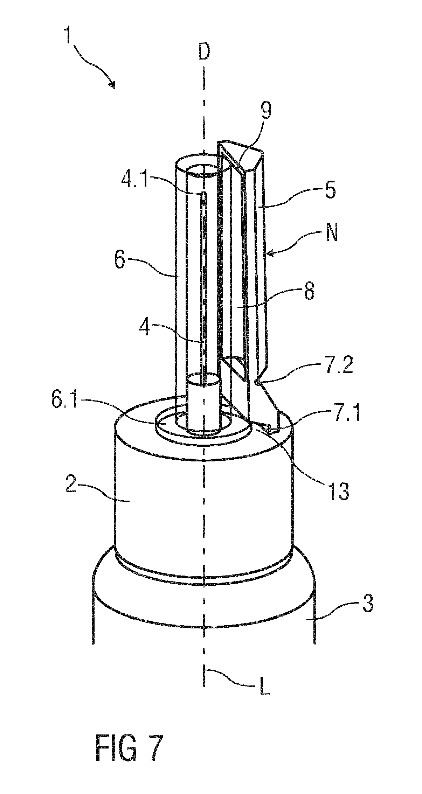

Figure 5 is a schematic transparent view of another exemplary embodiment of a needle assembly 1 according to the present invention prior to use. Figure 6 shows the needle assembly 1 after removal of a sterility tab 12. In an exemplary embodiment the needle assembly 1 comprises:
- a needle hub 2 adapted to be connected to a distal end of a medicament cartridge or a cartridge holder 3 for a medicament cartridge,
- a hollow needle 4 (cf. fig. 7) attached to the needle hub 2, the hollow needle 4 comprising a distal pointed tip 4.1,
- a needle catch arm 5 attached to the needle hub 2 and adapted to be tilted toward the needle 4 for covering the distal pointed tip 4.1.

In an exemplary embodiment the needle catch arm 5 extends from the needle hub 2 in a distal direction D in a neutral position N, e. g. substantially in parallel with the needle 4 , wherein a protective needle sheath 6 is removably arranged on the needle hub 2 radially inward of the needle catch arm 5 with respect to a longitudinal axis L for covering the needle 4 prior to use. The protective needle sheath 6 comprises an annular proximal collar 6.1 having a greater diameter than the tubular part of the protective needle sheath 6.

In an exemplary embodiment the needle catch arm 5 is connected to the needle hub 2 by a first live hinge 7.1 which is arranged such that in the neutral position N a tangential slot 13 is formed at a proximal end of the needle catch arm 5 radially inward of the first live hinge 7.1, wherein the annular proximal collar 6.1 of the protective needle sheath 6 engages in the slot 13 such that the protective needle sheath 6 cannot be moved in the distal direction D without tilting the needle catch arm 5 away from the needle 4.

In an exemplary embodiment a second live hinge 7.2 is arranged distally from the first live hinge 7.1 adapted to allow for tilting the catch arm 5 toward the needle 4.

In an exemplary embodiment the needle catch arm 5 comprises a recess 8 for receiving the needle 4, wherein the recess 8 is limited by a distal end wall 9 arranged to catch the distal pointed tip 4.1 of the needle 4.

In an exemplary embodiment the needle assembly 1 further comprises a needle cap 10 removably arranged over the needle hub 2 for covering the needle 4 and the needle catch arm 5.

In an exemplary embodiment the needle cap 10 comprises a longitudinal slot 11 in an outer wall of the needle cap 10 extending to a proximal opening in the needle cap 10. The longitudinal slot 11 has a width at least as great as a width of the needle catch arm 5.

In an exemplary embodiment a sterility tab 12 may be removably arranged over the longitudinal slot 11 prior to use indicating an unused needle assembly 1 when intact. The sterility tab 12 may extend over both the longitudinal slot 11 and the proximal opening of the needle hub 2 thus keeping the entire needle assembly 1 sterile prior to use.

In figure 5 the needle assembly 1 is in a state as delivered prior to use. The protective needle sheath 6 is in place over the needle 4, the needle catch arm 5 is in the neutral position N and the needle cap 10 is arranged over the needle hub 2, the protective needle sheath 6 and the needle catch arm 5. The sterility tab 12 is in place over the longitudinal slot 11.

In an exemplary embodiment, in order to prepare the needle assembly 1 for an injection the needle assembly 1 is attached to a distal end of a cartridge holder 3 or medicament cartridge.

The user may peel the sterility tab 12 off exposing the longitudinal slot 11 at this point or at any other point during operation. Figure 6 shows the needle assembly 1 after removal of the sterility tab 12.

The needle cap 10 may then be gripped and pulled in the distal direction D off the needle hub 2 thus exposing the needle catch arm 5 and the protective needle sheath 6. This state of the needle assembly 1 is illustrated in figure 7.

The protective needle sheath 6 may then be pulled off the needle 4 in the distal direction D exposing the needle 4 and rendering the needle assembly 1 ready to be used for an injection. Due to the annular collar 6.1 engaged in the slot 13 the needle catch arm 5 is tilted about the first live hinge 7.1 from the neutral position N towards a medicament delivery position MD when removing the protective needle sheath 6.

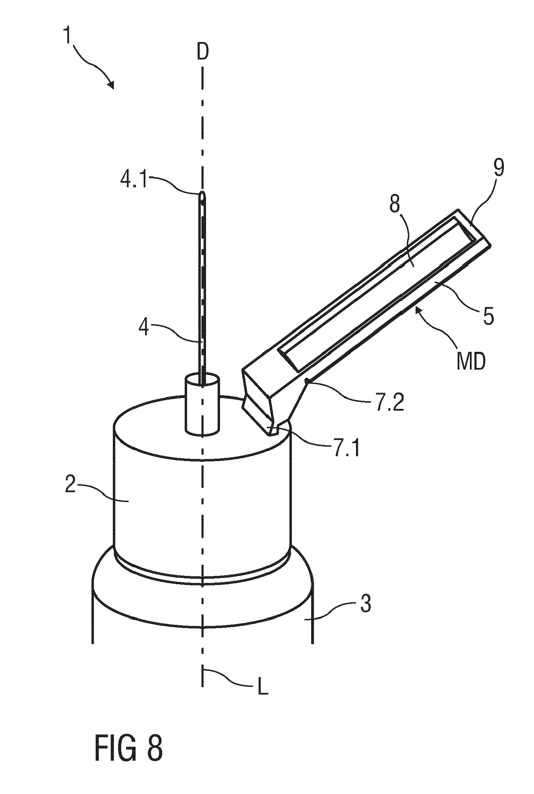

Figure 8 shows the needle assembly 1 with the needle catch arm 5 in the medicament delivery position MD, in which the needle catch arm 5 is tilted radially outward from the neutral position N thus facilitating insertion of the needle 4 into an injection site.

The needle 4 may now be inserted into an injection site and a medicament may be displaced from the medicament cartridge through a lumen in the needle 4.

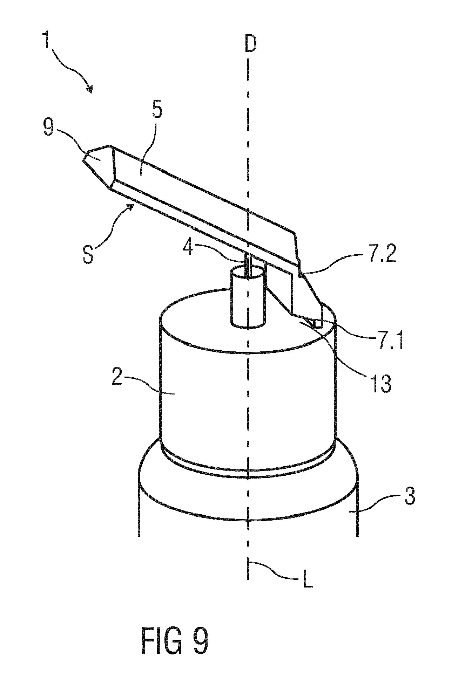

Subsequently or at any other point in time the needle catch arm 5 may be brought into a needle safe position S in which the needle 4 can no longer be accessed. Figure 9 shows the needle assembly 1 with the needle catch arm 5 in the needle safe position S, in which the needle catch arm 5 is tilted toward the needle 4 for covering the distal pointed tip 4.1 of the needle 4. In an exemplary embodiment a user may bring the needle catch arm 5 from the neutral N or delivery position MD into the needle safe position S by pressing the needle catch arm 5 against a surface, e. g. a table.

When the needle catch arm 5 is tilted into the needle safe position S the needle 4 is received within the recess 8. The distal end wall 9 prevents the distal pointed tip 4.1 of the needle 4 from protruding beyond the distal end of the needle catch arm 5.

In an exemplary embodiment the needle catch arm 5 is arranged to be tilted toward the needle 4 to such an extent that the needle 4 is plastically deformed, e.g. bent by the needle catch arm 5. In an exemplary embodiment the distal end wall 9 limiting the recess 8 within the needle catch arm 5 engages the distal pointed tip 4.1 of the needle 4 while the needle catch arm 5 is being tilted towards the needle safe position S and the needle 4 is bent within the recess 8. This results in the needle 4 being at least slightly tensioned within the recess 8 such that the distal pointed tip 4.1 may ingress into the material of the distal end 9 wall further improving post injection needle safety. The material of the distal end wall 9 may be particularly soft for improving this effect.

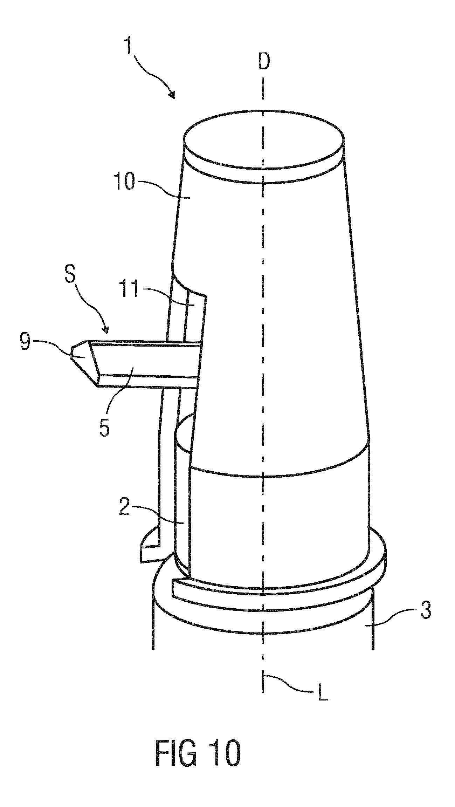

Subsequently the needle cap 10 is replaced over the needle hub 2. Figure 10 shows the needle assembly 1 with the re-attached needle cap 10. After re-attaching the needle cap 10 the needle catch arm 5 protrudes radially through the longitudinal slot 11 providing a clear indication that the needle assembly 1 has been used. If the sterility tab 12 has not been removed prior to removal of the needle cap 10 as in figure 6 it may likewise be removed prior to replacing the needle cap 10 over the needle hub 2.

The needle assembly 1 may now be removed from the cartridge holder 3 or medicament cartridge and disposed.

In all illustrated embodiments the needle cap 10 may comprise grip features (not illustrated) such as corrugations for improving ergonomics. Furthermore, the needle catch arm 5 and the needle hub 2 may comprise corresponding locking features (not illustrated) locking the needle catch arm 5 in the needle safe position S thus preventing re-exposure of the needle 4.

In an exemplary embodiment the needle hub 2 may comprise a screw thread (not illustrated) adapted to engage a corresponding screw thread on the medicament cartridge or cartridge holder 3. The screw thread may be arranged on an inner surface of a cavity within the needle hub 2. Likewise, the screw thread may be on an outer surface of the needle hub 2 and the medicament cartridge or cartridge holder 3 may have a corresponding thread on an inner surface. In other embodiments the needle hub 2 and the medicament cartridge or cartridge holder 3 may comprise corresponding bayonet features or they may have conical surfaces for frictionally fitting the needle hub 2 to the medicament cartridge or cartridge holder 3 (not illustrated).

In an exemplary embodiment the needle 4 may further comprise a proximal pointed tip (not illustrated) extending within a cavity of the needle hub 2 for establishing a fluid communication between the needle 4 and the medicament cartridge by the proximal pointed tip piercing a septum on the cartridge while the needle assembly 1 is being attached to the medicament cartridge.

Those of skill in the art will understand that modifications (additions and/or removals) of various components of the apparatuses, methods and/or systems and embodiments described herein may be made without departing from the full scope and spirit of the present invention, which encompass such modifications and any and all equivalents thereof.

## Claims

1. Needle assembly (1) comprising:
- a needle hub (2) adapted to be connected to a distal end of a medicament cartridge or cartridge holder (3),
- a hollow needle (4) attached to the needle hub (2), the hollow needle (4) comprising a distal pointed tip (4.1),
- a needle catch arm (5) attached to the needle hub (2) and adapted to be tilted toward the needle (4) for covering the distal pointed tip (4.1).

2. Needle assembly (1) according to claim 1, wherein the needle catch arm (5) extends from the needle hub (2) in a distal direction (D) in a neutral position (N), wherein a protective needle sheath (6) is removably arrangeable on the needle hub (2) radially inward of the needle catch arm (5) for covering the needle (4).

3. Needle assembly (1) according to one of the preceding claims, wherein the needle catch arm (5) is connected to the needle hub (2) by at least one live hinge (7.1, 7.2) adapted to allow for tilting the needle catch arm (5) toward and away from the needle (4).

4. Needle assembly (1) according to claims 2 and 3, wherein the protective needle sheath (6) comprises an annular proximal collar (6.1), wherein at least one of the live hinges (7.1, 7.2) is arranged to form a slot at a proximal end of the needle catch arm (5), wherein the annular proximal collar (6.1) is adapted to engage in the slot (13).

5. Needle assembly (1) according to one of the preceding claims, wherein the needle catch arm (5) comprises a recess (8) for receiving the needle (4), the recess (8) limited by a distal end wall (9) arranged to catch the distal pointed tip (4.1) of the needle (4).

6. Needle assembly (1) according to one of the preceding claims, wherein the needle catch arm (5) is arranged to be tilted toward the needle (4) to such an extent that the needle (4) is plastically deformed by the needle catch arm (5).

7. Needle assembly (1) according to one of the preceding claims, further comprising a needle cap (10) removably arrangeable over the needle hub (2) for covering the needle (4) and the needle catch arm (5).

8. Needle assembly (1) according to claim 7, wherein the needle cap (10) comprises a longitudinal slot (11) having a width at least as great as a width of the needle catch arm (5).

9. Needle assembly (1) according to claim 8, wherein a sterility tab (12) is removably arranged over the longitudinal slot (11).

10. Needle assembly (1) according to one of the preceding claims, wherein the needle hub (2) comprises a screw thread adapted to engage a corresponding screw thread on a medicament cartridge or cartridge holder (3).

11. Needle assembly (1) according to claim 10, wherein the screw thread is arranged on an inner surface of a cavity within the needle hub (2).

12. Needle assembly (1) according to one of the preceding claims, wherein the needle (4) comprises a proximal pointed tip extending within a cavity of the needle hub (2).

13. Needle assembly (1) according to one of the preceding claims, wherein the needle catch arm (5) and the needle hub (2) comprise corresponding locking features for locking the needle catch arm (5) in a needle safe position (S) covering the needle (4).

14. Needle assembly (1) according to one of the claims 2 to 13, wherein the protective needle sheath (6) and the needle catch arm (5) comprise corresponding features interacting in such a way that the needle catch arm (5) is tilted from the neutral position (N) towards a medicament delivery position (MD), in which the needle catch arm (5) is tilted outward from the neutral position (N), by removing the protective needle sheath (6) from the needle hub (2).
